(19) Europäisches Patentamt
European Patent Office
Office européen des brevets

(11) Publication number: **0 551 518 A1**

(12) **EUROPEAN PATENT APPLICATION published in accordance with Art. 158(3) EPC**

(21) Application number: **91917059.7**

(22) Date of filing: **02.10.91**

(86) International application number: **PCT/JP91/01327**

(87) International publication number: **WO 92/06099 (16.04.92 92/09)**

(51) Int. Cl.$^5$: **C07D 513/04**, A61K 31/47

(30) Priority: **03.10.90 JP 266003/90**

(43) Date of publication of application: **21.07.93 Bulletin 93/29**

(84) Designated Contracting States: **AT BE CH DE DK ES FR GB GR IT LI LU NL SE**

(71) Applicant: **NIPPON SHINYAKU COMPANY, LIMITED**
**14, Kisshoin Nishinosho Monguchicho**
**Minami-ku Kyoto-shi Kyoto 601(JP)**

(72) Inventor: **SEGAWA, Jun, 4-16, Suzaku 1-chome**
**Nara-shi**
**Nara 631(JP)**
Inventor: **KITANO, Masahiko, 9-4, Matsugasaki Donouecho**
**Sakyo-ku**
**Kyoto-shi Kyoto 606(JP)**
Inventor: **TOMII, Yoshifumi, 19-10, Hoshida 1-chome**
**Katano-shi**
**Osaka 576(JP)**

(74) Representative: **Kügele, Bernhard et al**
**NOVAPAT-CABINET CHEREAU 63 bis, boulevard Bessières**
**F-75017 Paris (FR)**

(54) **OUINOLINECARBOXYLIC ACID DERIVATIVE.**

(57) A novel quinolinecarboxylic acid derivative represented by general formula (I) and physiologically acceptable salt thereof, which are efficacious in treating various infectious diseases, particularly those caused by gram-positive cocci including methicillin-resistant staphylococcus and neuquinolone- and methicillin-resistant staphylococcus, wherein Z represents (un)substituted phenyl; $R^1$ represents hydrogen, alkyl or (un)substituted phenyl; $R^2$ represents hydrogen, alkoxy or halogen; and $R^3$ represents hydrogen or (un)substituted alkyl.

EP 0 551 518 A1

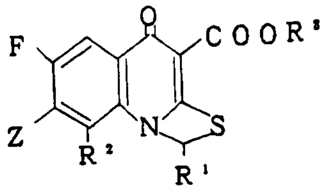

(I)

TECHNICAL FIELD

The present invention relates to a novel quinolinecarboxylic acid derivative which is of value as a therapeutic drug for various infectious diseases and to a synthetic intermediate thereof.

More particularly, the present invention relates to a novel quinolinecarboxylic acid derivative which is particularly useful for the treatment of infectious diseases caused by gram-positive bacteria including methicillin-resistant Staphylococcus aureus (MRSA) and new quinolone-resistant and methicillin-resistant Staphylococcus aureus (MRSA, PCA$^r$).

Still more particularly, the invention relates to a quinolinecarboxylic acid derivative of the following general formula and a physiologically acceptable salt thereof.

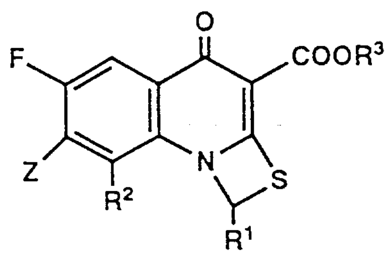

[I]

wherein Z is substituted or unsubstituted phenyl,
$R^1$ is hydrogen, alkyl, or substituted or unsubstituted phenyl,
$R^2$ is hydrogen, alkoxy or halogen,
$R^3$ is hydrogen or substituted or unsubstituted alkyl.

BACKGROUND ART

As synthetic antimicrobial agents for the treatment of bacterial infections, nalidixic acid, piromidic acid, pipemidic acid, enoxacin (AT-2266) and ofloxacin (DL-8280), among others, are commonly employed today.

However, these drugs are primarily active against gram-negative bacteria and not effective enough in the treatment of infectious diseases caused by gram-positive bacteria, particularly the diseases caused by methicillin-resistant S. aureus or new quinolone-resistant and methicillin-resistant S. aureus which are refractory to treatment and gaining in the frequency of occurrence.

The most effective drug against methicillin-resistant S. aureus which is currently available is said to be vancomycin, an antibiotic drug.

However, since this drug is a very high nephrotoxic potential, there is a constant risk of serious adverse reaction.

Meanwhile, recent years have witnessed the emergence of new quinolone-resistant methicillin-resistant S. aureus which is resistant even to new quinolone synthetic antimicrobial agents and the incidence of hospital infection associated with this organism is presenting a social problem.

Thus, there is not available a therapeutic drug suited for the treatment of infectious diseases caused by methicillin-resistant S. aureus or new quinolone-resistant and methicillin-resistant S. aureus.

Meanwhile, 6-fluorothiazetoquinolinecarboxylic acid derivatives have been disclosed in Japanese Patent (JP) Kokai No. 63-107990, JP Kokai No. 1-294680 and JP Kokai No. 1-230584, for instance.

However, none of the claimed compounds have a phenyl group at C-7, nor any of the above patent literature include even a suggestion about whether these compounds are active or not against methicillin-resistant S. aureus or new quinolone-resistant and methicillin-resistant S. aureus.

New quinolone antimicrobial agents having a 7-phenyl group are disclosed in JP Kokai 61-143365 and JP Kokai 1-319463.

However, none of the claimed compounds are 6-fluorothiazetoquinolinecarboxylic acid derivatives, nor these patent literature include even a suggestion about whether these compounds are active against methicillin-resistant S. aureus or new quinolone-resistant and methicillin-resistant S. aureus.

DISCLOSURE OF INVENTION

It is, therefore, the object of the present invention to provide a novel quinolinecarboxylic acid derivative which is still more effective than the quinolinecarboxylic acid derivative which the present applicant

previously disclosed as an antimicrobial agent which is effective in the treatment of various infectious diseases caused by, inter alia, by gram-positive bacteria including of methicillin-resistant S. aureus and new quinolone-resistant and methicillin-resistant S. aureus, and particularly active against methicillin-resistant S. aureus (WO 91-/07412).

To accomplish the above object, the inventors of the present invention synthesized and screened a large number of 6-fluorothiazetoquinolinecarboxylic acid derivatives which might be effective for various infectious diseases and, in so doing, surprisingly discovered that 6-fluorothiazetoquinolinecarboxylic acid derivatives having a phenyl group at C-7 are remarkably active against methicillin-resistant S. aureus and new quinolone-resistant and methicillin-resistant S. aureus. They accordingly brought the present invention into being.

The gist of the present invention is therefore, concerned with 6-fluorothiazetoquinolinecarboxylic acid derivatives substituted with optionally substituted phenyl group at C-7. The present invention is now described in detail.

Referring to general formula [I], Z is substituted or unsubstituted phenyl. The substituent of substituted phenyl Z may for example be hydroxy, alkoxy, hydroxyalkyl, alkoxyalkyl, nitro, halogen,

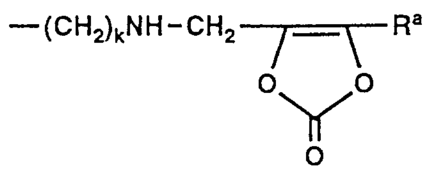

or

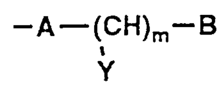

(k is 0 or 1, $R^a$ is alkyl, A is a bond or $-(CH_2)nNHCO-$, n is 0 or 1, Y is hydrogen or hydroxy, m is 0 or 1, -B is $-CH_3$ or

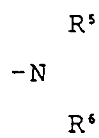

,

wherein $R^5$, $R^6$ may be the same or different and each is hydrogen or alkyl].

Among these groups, the substituent group with its nitrogen atom directly bound to the phenyl is preferred to the remainder.

The alkoxy as the substituent of said phenyl is preferably a straight-chain or branched lower alkoxy group of 1 to 4 carbon atoms, such as methoxy, ethoxy, n-propoxy, isopropoxy, n-butoxy, isobutoxy, sec-butoxy or tert-butoxy.

The hydroxyalkyl as the substituent of said phenyl is preferably a group having 1 to 4 carbon atoms, such as hydroxymethyl, hydroxyethyl, hydroxypropyl or hydroxybutyl.

The alkoxyalkyl as the substituent of said phenyl is preferably a group of 2 to 6 carbon atoms, such as methoxymethyl, methoxyethyl, methoxypropyl, methoxybutyl, ethoxymethyl, ethoxyethyl, ethoxypropyl, ethoxybutyl, propoxymethyl, propoxyethyl or propoxypropyl.

The halogen as the substituent of said phenyl may for example be fluorine, chlorine, bromine or iodine.

The alkyl represented by $R^1$, $R^5$, $R^6$, $R^a$ is preferably a straight-chain or branched alkyl group of 1 to 4 carbon atoms, such as methyl, ethyl, n-propyl, isopropyl, n-butyl, isobutyl, sec-butyl and tert-butyl.

Aside from the above, the substituent of said phenyl may be any of the amino, alkylamino or aminoalkyl group to which a group having the function of forming a prodrug has been attached.

$R^1$ is hydrogen, alkyl, or optionally substituted phenyl, although hydrogen and alkyl are particularly preferred.

The substituted phenyl for $R^1$ may for example be halophenyl. This halogen and the halogen represented by $R^2$ may each be fluorine, chlorine, bromine or iodine, for instance.

$R^3$ is hydrogen or optionally substituted alkyl.

The alkyl represented by $R^3$ is preferably a straight-chain or branched alkyl group of 1 to 4 carbon atoms, such as methyl, ethyl, n-propyl, isopropyl, n-butyl, isobutyl, sec-butyl or tert-butyl.

The substituent of this alkyl may for example be pivaloyloxy, carbamoyl, mono- or dialkyl-substituted carbamoyl, or

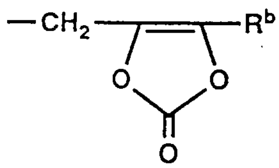

[$R^b$ is alkyl].

The alkyl which is a substituent of the nitrogen atom of said carbamoyl which is the substituent of said alkyl and the alkyl represented by $R^b$ each is preferably a straight-chain or branched alkyl group of 1 to 4 carbon atoms, such as methyl, ethyl, n-propyl, isopropyl, n-butyl, isobutyl, sec-butyl or tert-butyl.

Furthermore, $R^3$ may be a group to form a prodrug.

As the salt of compound [I] included in the scope of the invention, there may be mentioned salts with mineral acids such as hydrochloric acid, sulfuric acid, nitric acid, phosphoric acid, hydrofluoric acid and hydrobromic acid, salts with organic acids such as formic acid, acetic acid, tartaric acid, lactic acid, citric acid, fumaric acid, maleic acid, succinic acid, methanesulfonic acid, ethanesulfonic acid, benzenesulfonic acid, toluenesulfonic acid, naphthalenesulfonic acid and camphorsulfonic acid, and salts with alkali metals and alkaline earth metals such as sodium, potassium and calcium.

As examples of the compound of the present invention, the following compounds can be mentioned in addition to the compounds described in the examples given hereinafter in connection with the production processes.

(1) Ethyl 7-(4-aminophenyl)-6-fluoro-4-oxo-4H-[1,3]thiazeto[3,2-a]quinoline-3-carboxylate
(2) Ethyl 7-(4-aminomethylphenyl)-6-fluoro-4-oxo-4H-[1,3]thiazeto[3,2-a]quinoline-3-carboxylate
(3) 7-(4-Aminomethylphenyl)-6-fluoro-4-oxo-4H-[1,3]thiazeto[3,2-a]quinoline-3-carboxylic acid
(4) Ethyl 7-(4-aminomethylphenyl)-6,8-difluoro-4-oxo-4H-[1,3]thiazeto[3,2-a]quinoline-3-carboxylate
(5) 7-(4-Aminomethylphenyl)-6,8-difluoro-4-oxo-4H-[1,3]thiazeto[3,2-a]quinoline-3-carboxylic acid
(6) Ethyl 7-(4-aminomethylphenyl)-6-fluoro-8-methoxy-4-oxo-4H-[1-,3]thiazeto[3,2-a]quinoline-3-carboxylate
(7) 6-Fluoro-1-methyl-4-oxo-7-phenyl-4H-[1,3]thiazeto[3,2-a]quinoline-3-carboxylic acid
(8) 7-(4-Aminophenyl)-6-fluoro-1-methyl-4-oxo-4H-[1,3]thiazeto[3,2-a]quinoline-3-carboxylic acid
(10) Ethyl 6-fluoro-1-methyl-7-(4-methylaminophenyl)-4-oxo-4H-[1,3]thiazeto[3,2-a]quinoline-3-carboxylate
(11) Ethyl 7-(4-dimethylaminophenyl)-6-fluoro-1-methyl-4-oxo-4H-[1,3]thiazeto[3,2-a]quinoline-3-carboxylate
(12) Ethyl 6-fluoro-1-methyl-7-(4-methylaminomethylphenyl)-4-oxo-4H-[1,3]thiazeto[3,2-a]quinoline-3-carboxylate
(13) 6-Fluoro-1-methyl-7-(4-methylaminomethylphenyl)-4-oxo-4H-[1,3]thiazeto[3,2-a]quinoline-3-carboxylic acid
(14) Ethyl 7-(4-dimethylaminomethylphenyl)-6-fluoro-1-methyl-4-oxo-4H-[1,3]thiazeto[3,2-a]quinoline-3-carboxylate
(15) 7-(4-Dimethylaminomethylphenyl)-6-fluoro-1-methyl-4-oxo-4H-[1,3]thiazeto[3,2-a]quinoline-3-carboxylic acid
(16) Ethyl 7-(4-aminomethylphenyl)-6,8-difluoro-1-methyl-4-oxo-4H-[1,3]thiazeto[3,2-a]quinoline-3-carboxylate
(17) 7-(4-Aminomethylphenyl)-6,8-difluoro-1-methyl-4-oxo-4H-[1,3]thiazeto[3,2-a]quinoline-3-carboxylic acid
(18) Ethyl 7-(4-aminomethylphenyl)-6-fluoro-8-methoxy-1-methyl-4-oxo-4H-[1,3]thiazeto[3,2-a]quinoline-3-carboxylate
(19) Ethyl 6-fluoro-4-oxo-1,7-diphenyl-4H-[1,3]thiazeto[3,2-a]quinoline-3-carboxylate
(20) 6-Fluoro-4-oxo-1,7-diphenyl-4H-[1,3]thiazeto[3,2-a]quinoline-3-carboxylic acid
(21) Ethyl 7-(4-aminophenyl)-6-fluoro-4-oxo-1-phenyl-4H-[1,3]thiazeto[3,2-a]quinoline-3-carboxylate
(22) Ethyl 7-(4-aminomethylphenyl)-6-fluoro-4-oxo-1-phenyl-4H-[1,3]thiazeto[3,2-a]quinoline-3-carboxylate
(23) 7-(4-Aminomethylphenyl)-6-fluoro-4-oxo-1-phenyl-4H-[1,3]thiazeto[3,2-a]quinoline-3-carboxylic acid
(24) Ethyl 6,8-difluoro-4-oxo-1,7-diphenyl-4H-[1,3]thiazeto[3,2-a]quinoline-3-carboxylate
(25) 6,8-Difluoro-4-oxo-1,7-diphenyl-4H-[1,3]thiazeto[3,2-a]quinoline-3-carboxylic acid
(26) Ethyl 7-(4-aminophenyl)-6,8-difluoro-4-oxo-1-phenyl-4H-[1,3]thiazeto[3,2-a]quinoline-3-carboxylate
(27) 7-(4-Aminophenyl)-6,8-difluoro-4-oxo-1-phenyl-4H-[1,3]thiazeto[3,2-a]quinoline-3-carboxylic acid
(28) Ethyl 7-(4-aminomethylphenyl)-6,8-difluoro-4-oxo-1-phenyl-4H-[1,3]thiazeto[3,2-a]quinoline-3-carboxylate

(29) 7-(4-Aminomethylphenyl)-6,8-difluoro-4-oxo-1-phenyl-4H-[1,3]thiazeto[3,2-a]quinoline-3-carboxylic acid
(30) Ethyl 7-(4-aminophenyl)-6-fluoro-8-methoxy-4-oxo-1-phenyl-4H-[1,3]thiazeto[3,2-a]quinoline-3-carboxylate
(31) Ethyl 7-(4-aminomethylphenyl)-6-fluoro-8-methoxy-4-oxo-1-phenyl-4H-[1,3]thiazeto[3,2-a]quinoline-3-carboxylate
(32) Ethyl 7-(4-aminophenyl)-6-fluoro-1-(4-fluorophenyl)-4-oxo-4H-[1,3]thiazeto[3,2-a]quinoline-3-carboxylate
(33) 7-(4-Aminophenyl)-6-fluoro-1-(4-fluorophenyl)-4-oxo-4H-[1,3]thiazeto[3,2-a]quinoline-3-carboxylic acid
(34) Ethyl 7-(4-aminomethylphenyl)-6-fluoro-1-(4-fluorophenyl)-4-oxo-4H-[1,3]thiazeto[3,2-a]quinoline-3-carboxylate
(35) 7-(4-Aminomethylphenyl)-6-fluoro-1-(4-fluorophenyl)-4-oxo-4H-[1,3]thiazeto[3,2-a]quinoline-3-carboxylic acid
(36) Ethyl 6-fluoro-1-(2-fluorophenyl)-4-oxo-7-phenyl-4H-[1,3]thiazeto[3,2-a]quinoline-3-carboxylate
(37) 6-Fluoro-1-(2-fluorophenyl)-4-oxo-7-phenyl-4H-[1,3]thiazeto[3,2-a]quinoline-3-carboxylic acid
(38) Ethyl 7-(4-aminophenyl)-6-fluoro-1-(2-fluorophenyl)-4-oxo-4H-[1,3]thiazeto[3,2-a]quinoline-3-carboxylate
(39) 7-(4-Aminophenyl)-6-fluoro-1-(2-fluorophenyl)-4-oxo-4H-[1,3]thiazeto[3,2-a]quinoline-3-carboxylic acid
(40) Ethyl 7-(4-aminomethylphenyl)-6-fluoro-1-(2-fluorophenyl)-4-oxo-4H-[1,3]thiazeto[3,2-a]quinoline-3-carboxylate
(41) 7-(4-Aminomethylphenyl)-6-fluoro-1-(2-fluorophenyl)-4-oxo-4H-[1,3]thiazeto[3,2-a]quinoline-3-carboxylic acid
(42) Ethyl 6-fluoro-1-(2,4-difluorophenyl)-4-oxo-7-phenyl-4H-[1,3]thiazeto[3,2-a]quinoline-3-carboxylate
(43) 6-Fluoro-1-(2,4-difluorophenyl)-4-oxo-7-phenyl-4H-[1,3]thiazeto[3,2-a]quinoline-3-carboxylic acid
(44) Ethyl 7-(4-aminophenyl)-6-fluoro-1-(2,4-difluorophenyl)-4-oxo-4H-[1,3]thiazeto[3,2-a]quinoline-3-carboxylate
(45) 7-(4-Aminophenyl)-6-fluoro-1-(2,4-difluorophenyl)-4-oxo-4H-[1,3]thiazeto[3,2-a]quinoline-3-carboxylic acid
(46) Ethyl 7-(4-aminomethylphenyl)-6-fluoro-1-(2,4-difluorophenyl)-4-oxo-4H-[1,3]thiazeto[3,2-a]quinoline-3-carboxylate
(47) 7-(4-Aminomethylphenyl)-6-fluoro-1-(2,4-difluorophenyl)-4-oxo-4H-[1,3]thiazeto[3,2-a]quinoline-3-carboxylic acid
(48) Ethyl 6-fluoro-7-(4-fluorophenyl)-1-methyl-4-oxo-4H-[1,3]thiazeto[3,2-a]quinoline-3-carboxylate
(49) 6-Fluoro-7-(4-fluorophenyl)-1-methyl-4-oxo-4H-[1,3]thiazeto[3,2-a]quinoline-3-carboxylic acid
(50) 7-(4-Aminoacetylaminophenyl)-6-fluoro-1-methyl-4-oxo-4H-[1,3]thiazeto[3,2-a]quinoline-3-carboxylic acid
(51) 6-Fluoro-7-[4-(2-hydroxypropionyl)aminophenyl]-1-methyl-4-oxo-4H-[1,3]thiazeto[3,2-a]quinoline-3-carboxylic acid
(52) 7-(4-Aminoacetylaminomethylphenyl)-6-fluoro-1-methyl-4-oxo-4H-[1,3]thiazeto[3,2-a]quinoline-3-carboxylic acid
(53) 6-Fluoro-7-[4-(2-hydroxypropionyl)aminomethylphenyl]-1-methyl-4-oxo-4H-[1,3]thiazeto[3,2-a]-quinoline-3-carboxylic acid
(54) 6-Fluoro-1-methyl-7-[4-(5-methyl-2-oxo-1,3-dioxolen-4-yl)methylaminomethylphenyl]-4-oxo-4H-[1,3]-thiazeto[3,2-a]quinoline-3-carboxylic acid
(55) Ethyl 6-fluoro-7-(4-hydroxyphenyl)-1-methyl-4-oxo-4H-[1,3]th-iazeto[3,2-a]quinoline-3-carboxylate
(56) Ethyl 6-fluoro-7-(4-methoxyphenyl)-1-methyl-4-oxo-4H-[1,3]thiazeto[3,2-a]quinoline-3-carboxylate
(57) Ethyl 6-fluoro-7-(4-hydroxymethylphenyl)-1-methyl-4-oxo-4H-[1,3]thiazeto[3,2-a]quinoline-3-carboxylate
(58) 6-Fluoro-7-(4-hydroxymethylphenyl)-1-methyl-4-oxo-4H-[1,3]thiazeto[3,2-a]quinoline-3-carboxylic acid
(59) Ethyl 6-fluoro-7-(4-methoxymethylphenyl)-1-methyl-4-oxo-4H-[1,3]thiazeto[3,2-a]quinoline-3-carboxylate
(60) 6-fluoro-7-(4-methoxymethylphenyl)-1-methyl-4-oxo-4H-[1,3]thiazeto[3,2-a]quinoline-3-carboxylic acid
(61) Pivaloyloxymethyl 6-fluoro-1-methyl-4-oxo-7-phenyl-4H-[1,3]thiazeto[3,2-a]quinoline-3-carboxylate

The compound of the present invention can be produced by the following and other processes.

Process A:

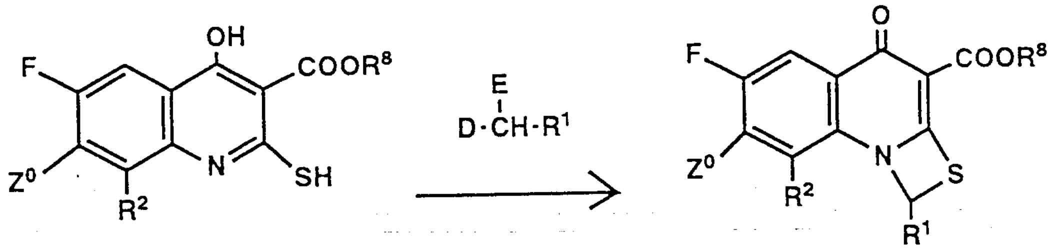


[II] [Ia]

wherein $R^1$ and $R^2$ are as defined hereinbefore; $R^8$ is alkyl; D and E are the same or different and each is halogen; $Z^0$ is optionally substituted phenyl.

The substituted phenyl represented by $Z^0$ may for example alkoxyphenyl, alkoxyalkylphenyl, nitrophenyl, halophenyl, protected aminophenyl or protected aminoalkylphenyl.

The alkyl represented by $R^8$, the alkyl of alkoxyalkylphenyl for $Z^0$ and the alkyl of protected aminoalkylphenyl for $Z^0$ are each preferably a straight-chain or branched alkyl group of 1 to 4 carbon atoms, such as methyl, ethyl, n-propyl, isopropyl, n-butyl, isobutyl, sec-butyl, tert-butyl, etc.

The alkoxy of alkoxyphenyl or alkoxyalkylphenyl represented by $Z^0$ is preferably a straight-chain or branched lower alkoxy group of 1 to 4 carbon atoms, such as methoxy, ethoxy, n-propoxy, isopropoxy, n-butoxy, isobutoxy, sec-butoxy, tert-butoxy, etc.

The halogen D, E and the halogen of halophenyl represented by $Z^0$ may for example be fluorine, chlorine, bromine or iodine.

The quinolinecarboxylic acid derivative [II] is reacted to cyclize with dihalide (e.g. methylene diiodide, ethylidene bromide, benzylidene bromide) in the presence of an acid acceptor (e.g. sodium carbonate, potassium carbonate, triethylamine) in an inert solvent, generally at 0 - 120 ° C, to give [Ia].

The solvent is preferably an aprotic solvent such as N,N-dimethylformamide, N,N-dimethylacetamide, dimethyl sulfoxide (DMSO) or sulfolane. The proportions of the dihalide and acid acceptor are not less than equimolar to [II] and preferably 1.1 - 2.5 mol equivalents on the same basis. To accelerate the reaction, 0.01 3.0 mol equivalents of sodium iodide or potassium iodide may be added to the reaction system.

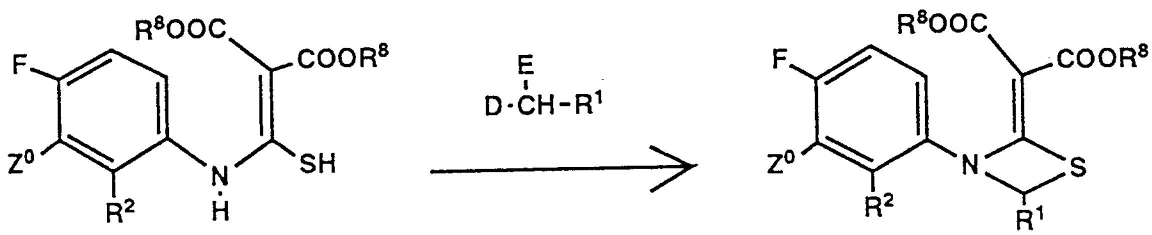


[III] [IV]

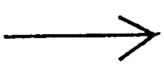 [Ia]

wherein $Z^0$, $R^1$, $R^2$, $R^8$, D and E are the same as defined under Process A.

Thus, [III] is reacted with dihalide in the presence of an acid acceptor (e.g potassium carbonate) in an inert solvent (e.g. N,N-dimethylformamide). Then, [IV] is cyclized to provide [Ia]. This cyclization reaction can be conducted in the per se known manner. For example, cyclization by heating or cyclization using an

acidic reagent such as phosphorus oxychloride, phosphorus pentachloride, phosphorus trichloride, thionyl chloride, concentrated sulfuric acid, polyphosphoric acid and a polyphosphoric ester can be mentioned. When the acidic reagent is used, for instance, the acidic reagent is used in a proportion of 1 mol equivalent to a large excess, preferably 20-30 mol equivalents, relative to [IV], and the reaction is conducted generally at 0 - 100 °C and preferably at 0 - 60 °C.

The compound having a substituted phenyl group at C-7 can also be produced by the steps of preparing [Ia] from [II] or [III] having a unsubstituted phenyl group in the above manner and then introducing the required substituent group by the per se known procedure.

If desired, the compound ($R^8$ is alkyl) obtained as above can be hydrolyzed to the carboxylic acid ($R^3$ is hydrogen). This hydrolysis is carried out using a large excess, preferably 10 - 20 equivalents, of an acid (e.g. sulfuric acid, hydrochloric acid, hydrobromic acid, hydrobromic acid-acetic acid, chlorosulfonic acid, polyphosphoric acid, etc.) at a temperature ranging from room temperature to 110 °C. The hydrolysis can also be effected by stirring a solution of the starting compound in aqueous alcohol [methanol, ethanol, propanol, butanol (preferably tert-butanol), etc.] containing 1 - 5% of potassium hydroxide or sodium hydroxide, which corresponds to 2 to 30 equivalents (preferably 5 to 10 equivalents), at room temperature to 60 °C. The protective group can be removed by the per se known procedure, if desired.

The ester can be converted to a different kind of ester by heating and stirring the starting material ester in 10 to 100 equivalents of the corresponding alcohol in the presence of a catalytic amount of concentrated sulfuric acid at 60 - 150 °C, preferably at 100 - 110 °C. The protective group can be removed, if desired, by the per se known procedure.

The carboxylic acid ($R^3$ is hydrogen) can be converted, if desired, to an ester ($R^3$ is optionally substituted alkyl group). This esterification reaction can be conducted by the per se known esterification procedure, for example using thionyl chloride and an alcohol, an alcohol and a condensing agent (e.g. dicyclohexylcarbodiimide), or an alkyl halide or substituted alkyl halide and an alcoholate. The carboxylic acid can also be converted to a pharmaceutically acceptable salt (e.g. with sodium or potassium) by the per se known procedure and used. The protective group can be removed, if desired, by the per se known procedure.

Among the compounds obtainable by the above-described processes, the compound having an aminophenyl or aminoalkylphenyl group at C-7 can be converted to an 5-alkyl-2-oxo-1,3-dioxol-4-ylmethylaminophenyl or 5-alkyl-2-oxo-1,3-dioxol-4-ylmethylaminoalkylphenyl compound by the reaction with the 5-alkyl-4-halomethyl-1,3-dioxol-2-one in the presence of a basic catalyst.

The resulting desired compound [I] can be isolated and purified by the per se known procedure such as concentration, pH adjustment, redistribution, solvent extraction, crystallization, recrystallization, fractional distillation and chromatography.

As will be described hereinafter, the starting compounds [II] and [III] can be prepared in accordance with the following reaction schemas.

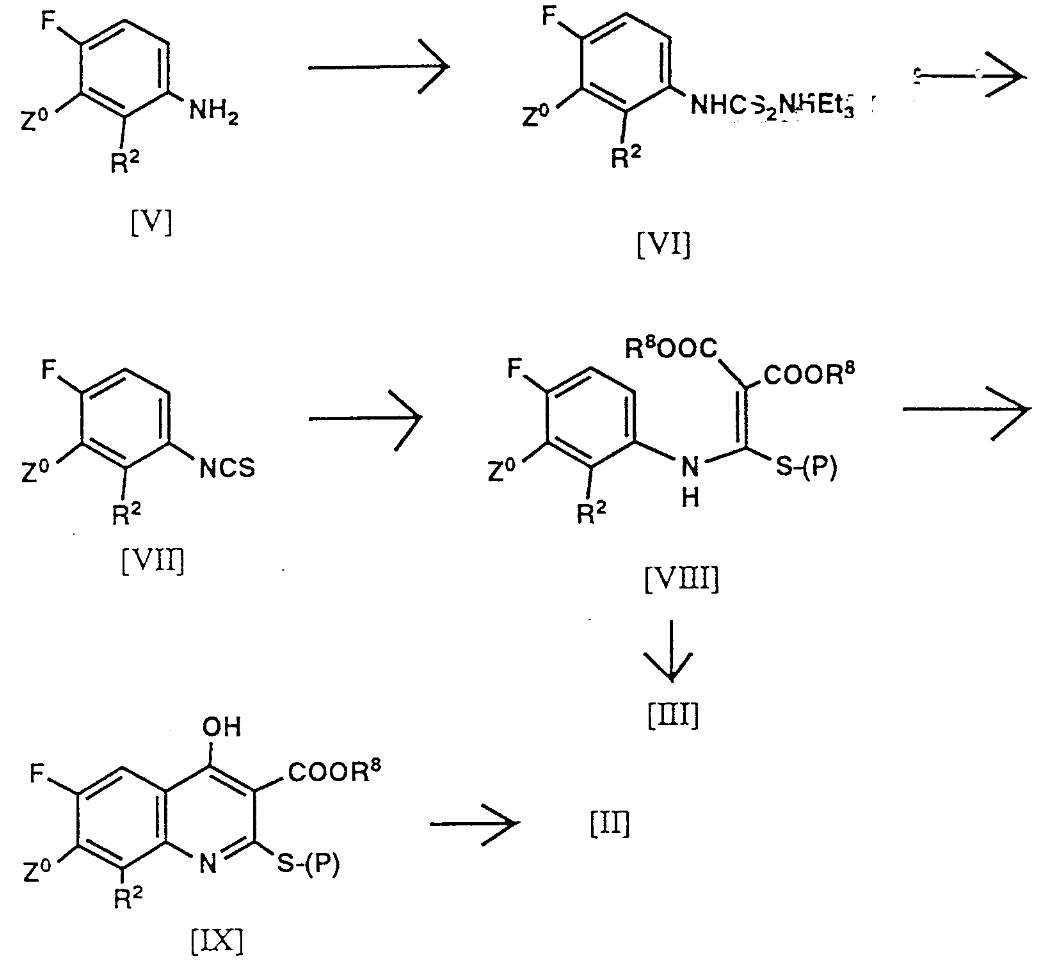

[V] [VI] [VII] [VIII] [III] [IX] [II]

(wherein $Z^0$, $R^2$ and $R^8$ are the same as defined hereinbefore; (P) is the protective group for S).

The aminobiphenyl [V] is reacted with carbon disulfide in the presence of a large excess of an amine such as triethylamine or an alkali metal either in a suitable solvent (e.g. benzene, chloroform, etc.) or in the absence of a solvent under cooling to provide a dithiocarbamic acid salt [VI]. This salt is reacted either with ethyl chlorocarbonate in the presence of triethylamine or with copper sulfate, lead nitrate, iron sulfate, zinc sulfide or the like in a suitable solvent (e.g. chloroform, methylene chloride) to provide the biphenyl isothiocyanate [VII]. Alternatively, [V] is reacted with thiophosgene in the presence of triethylamine in a suitable solvent (e.g. chloroform, THF, etc.) to give [VII].

This compound is reacted with dialkyl malonate sodium to give the biphenylaminomercaptomethylenemalonic acid dialkyl ester. The thiol group is protected to give [VIII] which is cyclized to [IX] under heating in a high-boiling solvent (e.g. dichlorobenzene, tetralin, diphenyl ether, diethylene glycol dimethyl ether, etc.). The protective group is then removed by the per se known procedure to give [II].

[III] is obtained by deprotecting [VIII] by the per se known procedure.

For use as a drug, the compound of the present invention is administered either as it is or as previously formulated with a pharmaceutically acceptable nontoxic, inert carrier and the resulting pharmaceutical composition containing 0.1 to 99.5%, preferably 0.5 to 90%, of the compound is administered to animals including of human.

The carrier may be at least one member of solid, semisolid or liquid diluents, fillers and other pharmaceutical auxiliary agents. The pharmaceutical composition is preferably administered in unit dosage forms. The pharmaceutical composition of the present invention can be administered orally, into the tissue, locally (e.g. transdermally) or rectally. Of course, the dosage form suitable for each route of administration should be selected. For example, oral administration is particularly preferred.

The dosage as a therapeutic drug for infectious disease is preferably adjusted according to the patient's age, body weight and other characteristics, the type and severity of disease and other conditions. Usually, however, the daily dosage as the active compound for human adults is 50 to 1000 mg/body, preferably 100 to 300 mg/body. A lower dosage may suffice or a higher dosage may be necessary, depending on cases. It

is also preferable that the above dosage is administered in 2 to 3 divided doses.

BEST MODE FOR CARRYING OUT THE INVENTION

The following reference examples and working examples concerning the production of the compound of the invention, and test examples of the compound of the invention are further descriptive of the present invention.

Reference Example 1 Synthesis of ethyl 6-fluoro-4-hydroxy-2-mercapto-7-phenylquinoline-3-carboxylate

(1) 2-Fluoro-5-isothiocyanatobiphenyl

In 20 ml of chloroform were dissolved 1.00 g of 5-amino-2-fluorobiphenyl and 0.65 g of triethylamine, and 0.82 g of thiophosgene was added dropwise at a temperature not exceeding 20 °C. The mixture was then stirred for 30 minutes, after which it was poured into a mixture of chloroform and saturated aqueous solution of sodium hydrogen carbonate. The chloroform layer was washed with saturated aqueous solution of sodium hydrogen carbonate and dried over magnesium sulfate and the solvent was distilled off to give an oil. This oil was not purified but directly used in the next reaction.

(2) Diethyl 6-fluoro-3-biphenylylaminomethoxymethylthiomethylenemalonate

In 20 ml of dry tetrahydrofuran (THF) was dissolved 1.03 g of diethyl malonate, and after 0.26 g of sodium hydride (60%) was added with ice-cooling, the mixture was allowed to return to room temperature. The mixture was then stirred for 30 minutes and a solution of 2-fluoro-5-isothiocyanatobiphenylin 10 ml of dry THF was added dropwise at a temperature not exceeding 10 °C. After this dropwise addition, the mixture was allowed to return to room temperature. The mixture was stirred for another 30 minutes, after which 0.43 g of chloromethyl methyl ether in 2 ml of dry THF was added dropwise with ice-cooling. The mixture was further stirred for 1 hour and the solvent was then distilled off. To the residue was added ice-water and the mixture was extracted with chloroform. The organic layer was washed with saturated aqueous solution of sodium chloride and dried over magnesium sulfate. The solvent was distilled off and the residue was purified by column chromatography ($SiO_2$, n-hexane-ethyl acetate = 20:1 → 5:1).
yield 2.06 g (86.2%)
IR (KBr) $\nu_{max}$ $cm^{-1}$: 2970, 1720, 1650, 1570, 1230, 1090, 695

(3) Ethyl 6-fluoro-4-hydroxy-2-methoxymethylthio-7-phenylquinoline-3-carboxylate

In 36.3 g of diphenyl ether was dissolved 12.10 g of diethyl 6-fluoro-3-biphenylylaminomethoxymethylthiomethylenemalonate and the solution was stirred on a hot water bath at 160 - 165 °C for 90 minutes. After cooling, the reaction mixture was purified by column chromatography ($SiO_2$, n-hexane-ethyl acetate = 20:1). The crystals obtained were recrystallized from isopropyl ether to provide 7.20 g of the title compound. m.p. 116-117 °C.

| Elemental analysis ($C_{20}H_{18}FNO_4S$) | | | |
|---|---|---|---|
| Calcd. (%) | C 62.00 | H 4.68 | N 3.62 |
| Found (%) | C 61.74 | H 4.80 | N 3.73 |

$^1$H-NMR ($CDCl_3$) ppm: 1.54 (3H, t, J = 7 Hz), 3.48 (3H, s), 4.57 (2H, q, J = 7 Hz), 5.49 (2H, s), 7.2-8.1 (5H, m), 7.33 (1H, d, J = 9 Hz), 7.91 (1H, d, J = 10.5 Hz), 13.20 (1H,s)

(4) Ethyl 6-fluoro-4-hydroxy-2-mercapto-7-phenylquinoline-3-carboxylate

To 200 ml of ethanol were added 6.67 g of ethyl 6-fluoro-4-hydroxy-2-methoxymethylthio-7-phenylquinoline-3-carboxylate and after 1 ml of concentrated sulfuric acid was added dropwise, the solution was refluxed for 60 minutes. The reaction mixture was then concentrated under reduced pressure and ice-cooled water was added to the residue. The resulting precipitate was collected by filtration, rinsed with water and dried under reduced pressure to provide 5.78 g of crystals. m.p. 211-212 °C (decomp.).
$^1$H-NMR ($CDCl_3$) ppm: 1.49 (3H, t, J = 7 Hz), 4.55 (2H, q, J = 7 Hz), 7.33 (1H, d, J = 8 Hz), 7.2-7.9 (5H, m),

7.85 (1H, d, J = 10.5 Hz)

Reference Example 2 Synthesis of ethyl 6-fluoro-4-hydroxy-2-mercapto-8-methoxy-7-phenylquinoline-3-carboxylate

(1) 6-Fluoro-2-hydroxy-3-nitrobiphenyl

In 6 ml of DMSO was dissolved 500 mg of 2,6-difluoro-3-nitrobiphenyl and after 6 ml of 10% aqueous solution of potassium hydroxide was added, the mixture was stirred on an oil bath at 50 °C for 30 minutes. The reaction mixture was then diluted with 10 ml of ice-water, made weakly acidic with 1% hydrochloric acid and extracted with ethyl acetate. The extract was washed with saturated aqueous solution of sodium chloride, dried and concentrated. The crystalline residue was purified by column chromatography ($SiO_2$ C-300, chloroform) to provide 390 mg of the title compound as yellow crystals. m.p. 106 °C.

(2) 6-Fluoro-2-methoxy-3-nitrobiphenyl

In 3 ml of DMF were dissolved 355 mg of potassium carbonate, 274 mg of methyl iodide and 300 mg of 6-fluoro-2-hydroxy-3-nitrobiphenyl and the solution was heated and stirred at 60 °C. The reaction mixture was then diluted with ice-water, made weakly acidic with 1% hydrochloric acid and extracted with ethyl acetate. The extract was washed with saturated aqueous solution of sodium chloride, dried over magnesium sulfate and concentrated to provide 300 mg of the title compound as an oil.

(3) 3-Amino-6-fluoro-2-methoxybiphenyl

In 6 ml of ethanol was dissolved 280 mg of 6-fluoro-2-methoxy-3-nitrobiphenyl and a solution of stannous chloride dihydrate (805 mg) in 3 ml of concentrated hydrochloric acid was slowly added dropwise with ice-cooling and stirring. The mixture was allowed to warm up to room temperature and stirred at room temperature for another 3 hours and at 50 °C for 1 hour. This reaction mixture was poured into ice-water, made basic with aqueous solution of sodium hydroxide and extracted with ethyl acetate. The extract was washed with saturated aqueous solution of sodium chloride, dried and concentrated to provide a crystalline residue. This residue was recrystallized from n-hexane to give 230 mg of the title compound. m.p. 75-76 °C.

(4) Ethyl 6-fluoro-4-hydroxy-2-mercapto-8-methoxy-7-phenylquinoline-3-carboxylate

Using 3-amino-6-fluoro-2-methoxybiphenyl, the procedure of Reference Example 1 was otherwise repeated to provide the title compound. m.p. 182-185 °C.

| Elemental analysis ($C_{19}H_{16}FNO_4S$) | | | |
|---|---|---|---|
| Calcd. (%) | C 61.12 | H 4.32 | N 3.75 |
| Found (%) | C 61.20 | H 4.39 | N 3.64 |

Reference Example 3 Synthesis of ethyl 6,8-difluoro- Synthesis of ethyl 6,8-difluoro-4-hydroxy-2-mercapto-7-phenylquinoline-3-carboxylate

(1) 3-Nitro-2,6-difluorobiphenyl

To a refluxed solution of 10 ml of n-amyl nitrite in 40 ml of benzene was added 5.00 g of 2,6-difluoro-3-nitroaniline in small portions and the mixture was further refluxed for 20 minutes. The reaction mixture was concentrated under reduced pressure and extracted with a few portions of hot n-hexane. The extract was concentrated under reduced pressure and the residue was subjected to column chromatography ($SiO_2$/n-hexane-ethyl acetate = 100:1 50:1) and recrystallized from n-hexane to provide 1.90 g of the title compound. m.p. 98.0 - 99.5 °C.

(2) 3-Amino-2,6-difluorobiphenyl

In 10 ml of ethanol was suspended 0.500 g of 3-nitro-2,6-difluorobiphenyl and a solution of 1.511 g of stannous chloride dihydrate in 4 ml of concentrated hydrochloric acid was added dropwise at a temperature not exceeding 15 °C. After the mixture was allowed to warm to room temperature, it was stirred for another 1 hour and heated at 60 for 1.5 hours. The ethanol was then distilled off and the residue was poured in ice-water, made basic with sodium hydroxide and extracted with ethyl acetate. The extract was dried and concentrated under reduced pressure to provide a crystalline residue. Recrystallization from n-hexane gave 279.1 mg of the title compound. m.p. 87-88 °C.

(3) Ethyl 6,8-difluoro-4-hydroxy-2-mercapto-7-phenylquinoline-3-carboxylate

Using 3-amino-2,6-difluorobiphenyl, the procedure of Reference Example 1 was otherwise repeated to give the title compound. m.p. 212-220 °C (decomp.)

| Elemental analysis ($C_{18}H_{13}F_2NO_3S$) | | | |
|---|---|---|---|
| Calcd. (%) | C 59.83 | H 3.63 | N 3.88 |
| Found (%) | C 59.77 | H 3.62 | N 3.76 |

Reference Example 4 Synthesis of ethyl 6-fluoro-7-(4-trifluoroacetamidophenyl)-4-hydroxy-2-mercaptoquinoline-3-carboxylate

2'-Fluoro-5'-nitrobiphenyl-4-carboxylic acid, a known compound, was subjected to Schmidt reaction in the known manner to give 4'-amino-2-fluoro-5-nitrobiphenyl which, then, was reacted with trifluoroacetamide and catalytically reduced in the known manner to give 5-amino-2-fluoro-4'-trifluoroacetamidobiphenyl.

Using this compound, the procedure of Reference Example 1 was otherwise repeated to give the title compound. m.p. 244 °C.

Reference Example 5 Synthesis of ethyl 7-(4-dimethylaminophenyl)-6-fluoro-4-hydroxy-2-mercaptoquinoline-3-carboxylate

4'-Amino-2-fluoro-5-nitrobiphenyl was reacted with formic acid and formal in to give the dimethylamino compound. Using this compound, the procedure of Reference Example 1 was otherwise repeated to give the title compound. m.p. 178 °C.

Reference Example 6 Synthesis of ethyl 6-fluoro-7-(4-trifluoroacetamidomethylphenyl)-4-hydrox-y-2-mercaptoquinoline-3-carboxylate

2-Fluoro-4'-(hydroxymethyl)-5-nitrobiphenyl, a known compound, was reacted with thionyl chloride to give 4'-(chloromethyl)-2-fluoro-5-nitrobiphenyl which, then, was reacted with trifluoroacetamide and catalytically reduced to give 5-amino-2-fluoro-4'-trifluoroacetamidomethylbiphenyl. Using this compound, the procedure of Reference Example 1 was otherwise repeated to give the title compound. m.p. 210-220 °C (decomp.)

Reference Example 7 Synthesis of ethyl 6-fluoro-7-(4-trifluoroacetamidophenyl)-4-hydroxy-2-me-rcapto-8-methoxyquinoline-3-carboxylate

2,6-Difluoroaniline, a known compound, was converted to diazonium tetrafluoroborate in the known manner and this salt was reacted with benzene to give the biphenyl compound. This compound was acetylated by Friedel-Crafts reaction using acetyl chloride to give 4'-acetyl-2,6-difluoro-3-nitrobiphenyl which, then, was treated with potassium hydroxide to give 4'-acetyl-6-fluoro-2-hydroxy-3-nitrobiphenyl. This compound was methylated with methyl iodide and further reacted with sodium hypobromite to give 6'-fluoro-2'-methoxy-3'-nitrobiphenyl-4-carboxylic acid. This compound was further reacted with thionyl chloride, then amidated with ammonia and subjected to Hoffmann's rearrangement in the known manner to give 4'-amino-6-fluoro-2-methoxy-3-nitrobiphenyl. Using this compound, the procedure described in Reference Example 4 and Reference Example 1 was repeated to give the title compound. m.p. 223 °C (decomp.)

| Elemental analysis ($C_{21}H_{16}F_4N_2O_5S$) | | | |
|---|---|---|---|
| Calcd. (%) | C 52.07 | H 3.33 | N 5.78 |
| Found (%) | C 52.40 | H 3.43 | N 5.76 |

Reference Example 8

(1) Synthesis of ethyl 6-fluoro-7-(4-trifluoroacetamidomethylphenyl)-4-hydrox-y-2-mercapto-8-methoxyquinoline-3-carboxylate

6'-Fluoro-2'-methoxy-3'-nitrobiphenyl-4-carboxylic acid was reduced with diborane in the known manner to give 6-fluoro-4'-(hydroxymethyl)-2-methoxy-3-nitrobiphenyl. Using this compound, the procedure of Reference Example 6 and Reference Example 1 was otherwise repeated to give the title compound. m.p. 191-193 °C (decomp.)

(2) Synthesis of ethyl 6-fluoro-4-hydroxy-2-mercapto-8-methoxy-7-[4-(N-methyltrifluoroacetamidomethyl)-phenyl]quinoline-3-carboxylate

Except that N-methyltrifluoroacetamide was used in lieu of trifluoroacetamide, the above process of synthesis was otherwise repeated to give the title compound. m.p. 168-169 °C (decomp.)

Reference Example 9

Synthesis of ethyl 6-fluoro-4-hydroxy-2-mercapto-7-(4-methoxyphenyl)quino-line-3-carboxylate

4'-Acetyl-2-fluorobiphenyl was nitrated in the known manner to give 4'-acetyl-2-fluoro-5-nitrobiphenyl which, in turn, was reacted with m-chloroperbenzoic acid and hydrolyzed to give 2-fluoro-4'-hydroxy-5-nitrobiphenyl. This compound was methylated with methyl iodide and the nitro group was then catalytically reduced in the known manner to give 5-amino-2-fluoro-4'-methoxybiphenyl. Using this compound, the procedure of Reference Example 1 was otherwise repeated to give the title compound. m.p. 240 °C.

| Elemental analysis ($C_{19}H_{16}FNO_4S$) | | | |
|---|---|---|---|
| Calcd. (%) | C 61.12 | H 4.32 | N 3.75 |
| Found (%) | C 61.25 | H 4.19 | N 3.65 |

Example 1 Ethyl 6-fluoro-1-methyl-4-oxo-7-phenyl-4H-[1,3]thiazeto[3,2-a]quinoline-3-carboxylate

To 6 ml of dry DMF were added 1.61 g of 1,1-dibromoethane, 1.61 g of potassium carbonate and 1.93 g of potassium iodide and the mixture was stirred at 95 °C for 30 minutes. To this mixture was added a solution of 2.00 g of the title compound of Reference Example 1 in 10 ml of dry DMF dropwise over a period of 1 hour and the mixture was stirred at the same temperature for 30 minutes. After cooling, the reaction mixture was poured into ice-water and extracted with chloroform. The extract was washed with 1% aqueous solution of potassium carbonate and, then, with saturated aqueous solution of sodium chloride and dried over magnesium sulfate. The solvent was distilled off under reduced pressure. The residue was recrystallized from ethyl acetate to give 1.19 g of the title compound. m.p. 218-219 °C (decomp.)

| Elemental analysis ($C_{20}H_{16}FNO_3S$) | | | |
|---|---|---|---|
| Calcd. (%) | C 65.03 | H 4.37 | N 3.79 |
| Found (%) | C 64.96 | H 4.99 | N 3.79 |

$^1$H-NMR δ ($CDCl_3$) ppm; 1.38 (3H, t, J = 7 Hz), 2.15 (3H, d, J = 6.5 Hz), 4.34 (2H, q, J = 7 Hz), 5.99 (1H, q, J = 6.5 Hz), 7.08 (1H, d, J = 6 Hz), 7.30 - 7.73 (5H, m), 8.11 (1H, d, J = 10.5 Hz)
IR (KBr) $\nu_{max}$ $cm^{-1}$; 1705, 1595, 1475, 1175, 1150, 765

Example 2

Ethy 6-fluoro-1-methyl-7-(4-nitrophenyl)-4-oxo-4H-[1,3]thia-zeto[3,2-a]quinoline-3-carboxylate

In 6 ml of concentrated sulfuric acid was dissolved 253.8 mg of the title compound of Example 1 with ice-cooling and after 104.2 mg of potassium nitrate was added in small portions, the mixture was further stirred at the same temperature for one hour. The reaction mixture was then poured into ice-water and extracted with chloroform-methanol. The extract was washed with saturated aqueous solution of sodium chloride and dried over magnesium sulfate and the solvent was distilled off under reduced pressure. The residue was recrystallized from ethyl acetate to give 165.3 mg of crystals. m.p. 252 °C (decomp.)

| Elemental analysis ($C_{20}H_{15}FN_2O_5S$) | | | |
|---|---|---|---|
| Calcd. (%) | C 57.97 | H 3.65 | N 6.76 |
| Found (%) | C 57.44 | H 4.22 | N 6.69 |

$^1$H-NMR ($CDCl_3$) ppm; 1.40 (3H, t, J = 7 Hz), 2.18 (3H, d, J = 6 Hz), 4.38 (2H, q, J = 7 Hz), 6.00 (1H, q, J = 6 Hz), 7.11 (1H, d, J = 6 Hz), 7.6-8.5 (4H, m), 8.20 (1H, d, J = 11 Hz)
IR (KBr) $\nu_{max}$ $cm^{-1}$ 3560, 1715, 1595, 1475, 1340, 1180, 850

Example 3 Ethyl 7-(4-aminophenyl)-6-fluoro-1-methyl-4-oxo-4H-[1,3]thiaz-eto[3,2-a]quinoline-3-carboxylate

In 50 ml of ethanol was suspended 201.8 mg of the title compound of Example 2 and a solution of 346.1 mg of stannous chloride dihydrate in 1.5 ml of concentrated sulfuric acid was added dropwise at a temperature not exceeding 55 °C. After 3 hours, a solution of 346.1 mg of stannous chloride dihydrate in 1.5 ml of concentrated sulfuric acid was further added and the mixture was stirred for another hour. The reaction mixture was then concentrated and poured into ice-water. The mixture was made basic with aqueous solution of sodium hydroxide and extracted with chloroform-methanol (5:1). The extract was washed with saturated aqueous solution of sodium chloride and dried over magnesium sulfate and the solvent was distilled off under reduced pressure. The residue was recrystallized from ethyl acetate to give 106.4 mg of the title compound. m.p. ≧ 300 °C.

| Elemental analysis ($C_{20}H_{17}FN_2O_3S$) | | | |
|---|---|---|---|
| Calcd. (%) | C 62.49 | H 4.46 | N 7.29 |
| Found (%) | C 62.39 | H 4.49 | N 7.21 |

$^1$H-NMR $\delta$ ($CDCl_3$) ppm; 1.57 (3H, t, J = 7 Hz), 2.43 (3H, d, J = 7 Hz), 4.71 (2H, q, J = 7 Hz), 6.71 (1H, q, J = 7 Hz), 7.72-8.12 (5H, m), 8.40 (1H, d, J = 10 Hz)
IR $\nu_{max}$ $cm^{-1}$: 410, 3320, 1705, 1595, 1475, 1340, 1280, 1085, 800

Example 4

7-(4-Aminophenyl)-6-fluoro-1-methyl-4-oxo-4H-[1,3]thia-zeto[3,2-a]quinoline-3-carboxylic acid

In a 5% solution of potassium hydroxide in a mixture of 8.5 ml of butanol and 3 ml of water was suspended 72.1 mg of the title compound of Example 3 and the suspension was stirred at 60 °C for 2 hours. After the tert-butanol was distilled off under reduced pressure, the residue was adjusted to pH 7 with diluted hydrochloric acid and extracted with chloroform-methanol (5:1). The extract was washed with saturated aqueous solution of sodium chloride and dried over magnesium sulfate and the solvent was distilled off under reeduced pressure. The residue was recrystallized from ethanol to give 41.2 mg of the title compound. m.p. 250 °C (decomp.)

| Elemental analysis ($C_{18}H_{13}FN_2O_3S\ H_2O$) | | | |
|---|---|---|---|
| Calcd. (%) | C 55.10 | H 4.37 | N 7.14 |
| Found (%) | C 55.53 | H 3.61 | N 7.11 |

$^1$H-NMR δ ($CDCl_3$) ppm; 2.44 (3H, d, J=7Hz), 6.70 (1H, q, J=7 Hz), 7.68-8.08 (5H, m), 8.41 (1H, d, J=10 Hz)
IR $\nu_{max}$ $cm^{-1}$: 3420, 3330, 1705, 1595, 1465, 1290, 785

Example 5 6-Fluoro-1-methyl-4-oxo-7-phenyl-4H-[1,3]thiazeto[3,2-a]quinoline-3-carboxylic acid

Using 300.0 mg of the title compound of Example 1, the procedure of Example 4 was otherwise repeated to give 181.9 mg of the title compound. m.p. 225-227°C (decomp.)

| Elemental analysis ($C_{18}H_{12}FNO_3S$) | | | |
|---|---|---|---|
| Calcd. (%) | C 63.33 | H 3.54 | N 4.10 |
| Found (%) | C 63.08 | H 3.99 | N 4.04 |

$^1$H-NMR δ ($CDCl_3$) ppm; 2.44 (3H, d, J=7 Hz), 6.65 (1H, q, J=7 Hz), 7.54-7.82 (4H, m), 7.86 (1H, d, J=6 Hz), 8.35 (1H, d, J=10 Hz)
IR $\nu_{max}$ $cm^{-1}$: 3030, 1695, 1595, 1465, 1085, 765

The following compounds were obtained in the same manner as Example 1.

Example 6 Ethyl 6-fluoro-4-oxo-7-phenyl-4H-[1,3]thiazeto[3,2-a]quinoline-3-carboxylate

m.p. ≧ 300°C

| Elemental analysis ($C_{19}H_{14}FNO_3S$ • 1/2 $H_2O$) | | | |
|---|---|---|---|
| Calcd. (%) | C 63.41 | H 4.06 | N 3.89 |
| Found (%) | C 63.15 | H 3.99 | N 3.73 |

IR $\nu_{max}$ $cm^{-1}$: 480, 1705, 1600, 1480

Example 7 Ethyl 6-Fluoro-1-(4-fluorophenyl)-4-oxo-7-phenyl-4H-[1,3]thiazeto[3,2-a]quinoline-3-carboxyate

m.p. 204°C
Mass spectrum $M^+$ :449

Example 8 Ethyl 6,8-difluoro-4-oxo-7-phenyl-4H-[1,3]thiazeto[3,2-a]quinoline-3-carboxylate

m.p. 257-263°C

| Elemental analysis ($C_{19}H_{13}FNO_3S$) | | | |
|---|---|---|---|
| Calcd. (%) | C 61.12 | H 3.51 | N 3.75 |
| Found (%) | C 61.39 | H 3.48 | N 3.80 |

Example 9 Ethyl 6-fluoro-8-methoxy-4-oxo-7-phenyl-4H-[1,3]thiazeto[3,2-a]quinoline-3-carboxylate

m.p. 272-274 °C

| Elemental analysis ($C_{20}H_{16}FNO_4S$) | | | |
|---|---|---|---|
| Calcd. (%) | C 62.33 | H 4.18 | N 3.63 |
| Found (%) | C 62.35 | H 3.94 | N 3.43 |

Example 10 Ethyl 6,8-difluoro-1-methyl-4-oxo-7-phenyl-4H-[1,3]thiazeto[3,2-a]quinoline-3-carboxylate

m.p. 212-213 °C (decomp.)

| Elemental analysis ($C_{20}H_{15}F_2NO_3S \cdot 1/2\ H_2O$) | | | |
|---|---|---|---|
| Calcd. (%) | C 60.60 | H 4.07 | N 3.53 |
| Found (%) | C 60.22 | H 3.80 | N 3.55 |

Example 11 Ethyl 6-fluoro-8-methoxy-1-methyl-4-oxo-7-phenyl-4H-[1,3]thiazeto[3,2-a]quinoline-3-carboxylate

m.p. 222-223 °C

| Elemental analysis (C21H18FNO4S) | | | |
|---|---|---|---|
| Calcd. (%) | C 63.15 | H 4.54 | N 3.51 |
| Found (%) | C 63.05 | H 4.77 | N 3.59 |

Example 12 Ethyl 6-fluoro-8-methoxy-4-oxo-1,7-diphenyl-4H-[1,3]thiazeto[3,2-a]quinoline-3-carboxylate

m.p. 181-182 °C

| Elemental analysis ($C_{26}H_{20}FNO_4S$) | | | |
|---|---|---|---|
| Calcd. (%) | C 67.67 | H 4.37 | N 3.03 |
| Found (%) | C 67.76 | H 4.27 | N 3.31 |

Example 13 Ethyl 7-(4-aminomethylphenyl)-6-fluoro-1-methyl-4-oxo-4H-[1,3]thiazeto[3,2-a]quinoline-3-carboxylate

m.p. 213-214 °C (decomp.)

| Elemental analysis ($C_{21}H_{19}FN_2O_3S$) | | | |
|---|---|---|---|
| Calcd. (%) | C 63.30 | H 4.81 | N 7.03 |
| Found (%) | C 63.30 | H 4.89 | N 7.09 |

The following compounds were obtained in the same manner as Example 2 and Example 3.

Example 14 Ethyl 7-(4-aminophenyl)-6,8-difluoro-4-oxo-4H-[1,3]thiazeto[3,2-a]quinoline-3-carboxylate
m.p. ≧ 300 ° C

| Elemental analysis ($C_{19}H_{14}F_2N_2O_3S$) | | | |
|---|---|---|---|
| Calcd. (%) | C 58.76 | H 3.63 | N 7.21 |
| Found (%) | C 58.68 | H 3.87 | N 7.02 |

IR $\nu_{max}$ $cm^{-1}$: 3350, 1690, 1600, 1480, 800

Example 15 Ethyl 7-(4-aminophenyl)-6-fluoro-8-methoxy-4-oxo-4H-[1,3]thiazeto[3,2-a]quinoline-3-carboxylate

m.p. 227-229 ° C (decomp.)

| Elemental analysis ($C_{20}H_{17}FN_2O_4S$ • 5/4 $H_2O$) | | | |
|---|---|---|---|
| Calcd. (%) | C 56.80 | H 4.65 | N 6.62 |
| Found (%) | C 56.82 | H 4.13 | N 6.67 |

Example 16 Ethyl 7-(4-aminophenyl)-6,8-difluoro-1-methyl-4-oxo-4H-[1,3]thiazeto[3,2-a]quinoline-3-carboxylate

m.p. ≧ 300 ° C

| Elemental analysis ($C_{20}H_{16}F_2N_2O_3S$) | | | |
|---|---|---|---|
| Calcd. (%) | C 59.69 | H 4.01 | N 6.96 |
| Found (%) | C 59.65 | H 4.16 | N 6.83 |

IR $\nu_{max}$ $cm^{-1}$: 3350, 1720, 1660, 1600, 1530, 1480

Example 17 Ethyl 7-(4-aminophenyl)-6-fluoro-8-methoxy-1-methyl-4-oxo-4H-[1,3]thiazeto[3,2-a]quinoline-3-carboxylate

m.p. 250-251 ° C (decomp.)

| Elemental analysis ($C_{21}H_{19}FN_2O_4S$) | | | |
|---|---|---|---|
| Calcd. (%) | C 60.86 | H 4.62 | N 6.76 |
| Found (%) | C 60.90 | H 4.56 | N 6.80 |

The following compounds were obtained in the same manner as Example 4.

Example 18 6-Fluoro-4-oxo-7-phenyl-4H-[1,3]thiazeto[3,2-a]quinoline-3-carboxylic acid

m.p. 224 ° C

| Elemental analysis ($C_{17}H_{10}FNO_3S$ • 1/4 $H_2O$) | | | |
|---|---|---|---|
| Calcd. (%) | C 61.53 | H 3.19 | N 4.22 |
| Found (%) | C 61.40 | H 3.33 | N 4.14 |

Example 19 6,8-Difluoro-4-oxo-7-phenyl-4H-[1,3]thiazeto[3,2-a]quinoline-3-carboxylic acid

m.p. 261-264 °C (decomp.)

| Elemental analysis ($C_{17}H_9F_2NO_3S$) | | | |
|---|---|---|---|
| Calcd. (%) | C 59.13 | H 2.63 | N 4.06 |
| Found (%) | C 59.08 | H 3.07 | N 4.02 |

Example 20 7-(4-Aminophenyl)-6,8-difluoro-4-oxo-4H-[1,3]thiazeto[3,2-a]quinoline-3-carboxylic acid

m.p. ≧ 300 °C

| Elemental analysis ($C_{17}H_{10}F_2N_2O_3S \cdot H_2O$) | | | |
|---|---|---|---|
| Calcd. (%) | C 53.97 | H 3.20 | N 7.40 |
| Found (%) | C 53.74 | H 3.20 | N 7.45 |

IR $\nu_{max}$ $cm^{-1}$: 3350, 1710, 1630, 1600, 1500, 1460

Example 21 6-Fluoro-8-methoxy-4-oxo-7-phenyl-4H-[1,3]thiazeto[3,2-a]quinoline-3-carboxylic acid

m.p. 265-267 °C (decomp.)

| Elemental analysis ($C_{18}H_{12}FNO_4S$) | | | |
|---|---|---|---|
| Calcd. (%) | C 60.50 | H 3.38 | N 3.92 |
| Found (%) | C 60.64 | H 3.36 | N 3.86 |

Example 22 7-(4-Aminophenyl)-6-fluoro-8-methoxy-4-oxo-4H-[1,3]thiazeto[3,2-a]quinoline-3-carboxylic acid
m.p. 248-260 °C (decomp.)

| Elemental analysis ($C_{18}H_{13}FN_2O_4S$) | | | |
|---|---|---|---|
| Calcd. (%) | C 58.06 | H 3.52 | N 7.52 |
| Found (%) | C 57.65 | H 3.40 | N 7.36 |

Example 23 7-(4-Aminomethylphenyl)-6-fluoro-1-methyl-4-oxo-4H-[1,3]thiazeto[3,2-a]quinoline-3-carboxylic acid

m.p. 213-214 °C (decomp.)

| Elemental analysis ($C_{19}H_{15}FN_2O_3S \cdot H_2O$) | | | |
|---|---|---|---|
| Calcd. (%) | C 58.75 | H 4.41 | N 7.21 |
| Found (%) | C 58.57 | H 5.07 | N 7.24 |

Example 24 6,8-Difluoro-1-methyl-4-oxo-7-phenyl-4H-[1,3]thiazeto[3,2-a]quinoline-3-carboxylic acid

m.p. 236-241 °C (decomp.)

| Elemental analysis ($C_{18}H_{11}F_2NO_3S$) | | | |
|---|---|---|---|
| Calcd. (%) | C 60.16 | H 3.09 | N 3.90 |
| Found (%) | C 60.03 | H 3.36 | N 3.94 |

Example 25 7-(4-Aminophenyl)-6,8-difluoro-1-methyl-4-oxo-4H-[1,3]thiazeto[3,2-a]quinoline-3-carboxylicacid

m.p. 230-235 °C (decomp.)

| Elemental analysis ($C_{18}H_{12}F_2N_2O_3S$) | | | |
|---|---|---|---|
| Calcd. (%) | C 57.75 | H 3.23 | N 7.48 |
| Found (%) | C 57.43 | H 3.44 | N 7.48 |

Example 26 6-Fluoro-8-methoxy-1-methyl-4-oxo-7-phenyl-4H-[1,3]thiazeto[3,2-a]quinoline-3-carboxylic acid
m.p. 239-240 °C (decomp.)

| Elemental analysis ($C_{19}H_{14}FNO_4S$) | | | |
|---|---|---|---|
| Calcd. (%) | C 61.45 | H 3.80 | N 3.77 |
| Found (%) | C 61.31 | H 3.97 | N 3.95 |

Example 27 7-(4-Aminophenyl)-6-fluoro-8-methoxy-1-methyl-4-oxo-4H[1,3]thiazeto[3,2-a]quinoline-3-carboxylic acid

m.p. 245-247 °C (decomp.)

| Elemental analysis ($C_{19}H_{15}FN_2O_4S$) | | | |
|---|---|---|---|
| Calcd. (%) | C 59.06 | H 3.91 | N 7.25 |
| Found (%) | C 58.98 | H 4.22 | N 7.13 |

Example 28 6-Fluoro-8-methoxy-4-oxo-1,7-diphenyl-4H-[1,3]thiazeto[3,2-a]quinoline-3-carboxylic acid

m.p. 184-185 °C

| Elemental analysis ($C_{24}H_{16}FNO_4S$) | | | |
|---|---|---|---|
| Calcd. (%) | C 66.50 | H 3.72 | N 3.23 |
| Found (%) | C 66.44 | H 3.66 | N 3.36 |

Example 29 6-Fluoro-1-(4-fluorophenyl)-4-oxo-7-phenyl-4H-[1,3]thi-azeto[3,2-a]quinoline-3-carboxylic acid

m.p. ≧ 300 ° C

| Elemental analysis ($C_{23}H_{13}F_2NO_3S$ • 1/2 $H_2O$) | | | |
|---|---|---|---|
| Calcd. (%) | C 64.18 | H 3.28 | N 3.25 |
| Found (%) | C 64.18 | H 3.15 | N 3.35 |

IR $\nu_{max}$ $cm^{-1}$: 1700, 1680, 1485, 1460

Example 30 7-(4-Dimethylaminophenyl)-6-fluoro-8-methoxy-1-methyl-4-oxo-4H-[1,3]thiazeto[3,2-a]quinoline-3-carboxylic acid

m.p. 237 - 238 ° C (decomp.)

| Elemental analysis ($C_{21}H_{19}FN_2O_4S$) | | | |
|---|---|---|---|
| Calcd. (%) | C 60.86 | H 4.62 | N 6.76 |
| Found (%) | C 60.74 | H 4.40 | N 6.80 |

The following compounds were obtained in the same manner as Example 1 and Example 4.

Example 31 7-(4-Aminophenyl)-6-fluoro-4-oxo-4H-[1,3]thiazeto[3,2-a]quinoline-3-carboxylic acid

m.p. ≧ 300 ° C

| Elemental analysis ($C_{17}H_{11}FN_2O_3S$ • 1/2 $H_2O$) | | | |
|---|---|---|---|
| Calcd. (%) | C 55.28 | H 3.82 | N 7.58 |
| Found (%) | C 55.02 | H 3.46 | N 7.33 |

IR $\nu_{max}$ $cm^{-1}$: 3350, 1690, 1600, 1480

Example 32 7-(4-Aminomethylphenyl)-6-fluoro-8-methoxy-4-oxo-4H-[1,3]thiazeto[3,2-a]quinoline-3-carboxylic acid

m.p. ≧ 300 ° C

| Elemental analysis (C19H15FN2O4S 2O) | | | |
|---|---|---|---|
| Calcd. (%) | C 56.43 | H 4.24 | N 6.93 |
| Found (%) | C 56.23 | H 4.30 | N 6.75 |

IR $\nu_{max}$ $cm^{-1}$: 3350, 1600, 1525, 1480

Example 33 7-(4-Dimethylaminophenyl)-6-fluoro-1- methyl-4-oxo-4H-[-1,3]thiazeto[3,2-a]quinoline-3-carboxylic acid

m.p. 237 ° C

| Elemental analysis ($C_{20}H_{17}FN_2O_3S$ • 3/4 $H_2O$) | | | |
|---|---|---|---|
| Calcd. (%) | C 60.37 | H 4.69 | N 7.04 |
| Found (%) | C 60.59 | H 4.48 | N 7.07 |

Example 34 7-(4-Aminomethylphenyl)-6-fluoro-8-methoxy-1-methyl-4-oxo-4H-[1,3]thiazeto[3,2-a]quinoline-3-carboxylic acid

m.p. ≧ 300 °C

| Elemental analysis ($C_{20}H_{17}FN_2O_4S$ • 2 $H_2O$) | | | |
|---|---|---|---|
| Calcd. (%) | C 55.04 | H 4.85 | N 6.42 |
| Found (%) | C 55.60 | H 4.78 | N 6.31 |

IR $\nu_{max}$ $cm^{-1}$: 3400, 2900, 1600, 1530, 1475

Example 35 7-(4-Aminophenyl)-6-fluoro-4-oxo-1-phenyl-4H-[1,3]thiazeto[3,2-a]quinoline-3-carboxylic acid

m.p. ≧ 300 °C

| Elemental analysis ($C_{23}H_{15}FN_2O_3S$ • 1/4 $H_2O$) | | | |
|---|---|---|---|
| Calcd. (%) | C 65.32 | H 3.69 | N 6.62 |
| Found (%) | C 65.37 | H 3.99 | N 6.31 |

IR $\nu_{max}$ $cm^{-1}$: 3450, 3350, 1700, 1620, 1600, 1485

Example 36 7-(4-Aminophenyl)-6-fluoro-8-methoxy-4-oxo-1-phenyl-4H-[1,3]thiazeto[3,2-a]quinoline-3-carboxylic acid

m.p. 228-229 °C (decomp.)

| Elemental analysis ($C_{24}H_{17}FN_2O_4S$ • 1/4 $H_2O$) | | | |
|---|---|---|---|
| Calcd. (%) | C 63.64 | H 3.89 | N 6.18 |
| Found (%) | C 63.70 | H 4.23 | N 6.18 |

Example 37 7-(4-Aminomethylphenyl)-6-fluoro-8-methoxy-4-oxo-1-phenyl-4H-[1,3]thiazeto[3,2-a]quinoline-3-carboxylic acid

m.p. ≧ 300 °C

| Elemental analysis ($C_{25}H_{19}FN_2O_4S$ • 2 $H_2O$) | | | |
|---|---|---|---|
| Calcd. (%) | C 60.23 | H 4.65 | N 5.62 |
| Found (%) | C 60.01 | H 4.59 | N 5.51 |

IR $\nu_{max}$ $cm^{-1}$: 1610, 1530, 1480

Example 38 6-Fluoro-7-(4-methoxyphenyl)-1-methyl-4-oxo-4H-[1,3]th-iazeto[3,2-a]quinoline-3-carboxylic acid

m.p. 242-246 °C (decomp.)

| Elemental analysis ($C_{19}H_{14}FNO_4S$) | | | |
|---|---|---|---|
| Calcd. (%) | C 61.45 | H 3.80 | N 3.77 |
| Found (%) | C 61.56 | H 3.90 | N 3.77 |

Example 39 7-(4-Aminomethylphenyl)-6-fluoro-1-(4-fluorophenyl)-8-methoxy-4-oxo-4H-[1,3]thiazeto[3,2-a]-quinoline-3-carboxylic acid

m.p. ≧ 300 °C

| Elemental analysis ($C_{25}H_{18}F_2N_2O_4S$ • 5/2 $H_2O$) | | | |
|---|---|---|---|
| Calcd. (%) | C 57.13 | H 4.41 | N 5.33 |
| Found (%) | C 56.86 | H 4.42 | N 5.38 |

IR $\nu_{max}$ $cm^{-1}$: 1600, 1530, 1510, 1440

Example 40 7-(4-Aminomethylphenyl)-6-fluoro-1-(2,4-difluorophenyl)-8-methoxy-4-oxo-4H-[1,3]thiazeto[3,2-a]quinoline-3-carboxylic acid

m.p. ≧ 300 °C

| Elemental analysis ($C_{25}H_{17}F_3N_2O_4S$ • 5/2 $H_2O$) | | | |
|---|---|---|---|
| Calcd. (%) | c 55.25 | H 4.08 | N 5.15 |
| Found (%) | C 55.26 | H 3.95 | N 4.97 |

IR $\nu_{max}$ $cm^{-1}$: 2900, 1600, 1530, 1475

The following compounds were also synthesized from the described compounds in the conventional manner.

Example 41 6-Fluoro-1-methyl-7-(4-methylaminophenyl)-4-oxo-4H-[1,3]thiazeto[3,2-a]quinoline-3-carboxylic acid

m.p. 215 °C (decomp.)

| Elemental analysis ($C_{19}H_{15}FN_2O_3S$ • 1/2 $H_2O$) | | | |
|---|---|---|---|
| Calcd. (%) | C 60.15 | H 4.25 | N 7.38 |
| Found (%) | C 60.39 | H 4.26 | N 7.14 |

Example 42 (5-Methyl-2-oxo-1,3-dioxolen-4-yl)methyl 6-fluoro-1-methyl-7-[4-(5-methyl-2-oxo-1,3-dioxolen-4-yl)methylaminophenyl]-4-oxo-4H-[1,3]thiazeto[3,2-a]quinoline-3-carboxylate

m.p. 236 °C (decomp.)

| Elemental analysis ($C_{28}H_{21}FN_2O_9S$) | | | |
|---|---|---|---|
| Calcd. (%) | C 57.93 | H 3.65 | N 4.83 |
| Found (%) | C 57.78 | H 3.70 | N 5.12 |

Example 43 6-Fluoro-1-methyl-7-[4-(5-methyl-2-oxo-1,3-dioxolen-4-yl)methylaminophenyl]-4-oxo-4H-[1,3]-thiazeto[3,2-a]quinoline-3-carboxylic acid

m.p. 242 °C (decomp.)

| Elemental analysis ($C_{23}H_{17}FN_2O_6S \cdot H_2O$) | | | |
|---|---|---|---|
| Calcd. (%) | C 56.79 | H 3.94 | N 5.75 |
| Found (%) | C 56.58 | H 3.68 | N 5.97 |

Example 44 6-Fluoro-7-(4-hydroxyphenyl)-1-methyl-4-oxo-4H-[1,3]thiazeto[3,2-a]quinoline-3-carboxylic acid
m.p. 284 °C (decomp.)

| Elemental analysis ($C_{18}H_{12}FNO_4S \cdot 1/4\ H_2O$) | | | |
|---|---|---|---|
| Calcd. (%) | C 59.75 | H 3.48 | N 3.87 |
| Found (%) | C 59.49 | H 3.53 | N 3.84 |

Example 45 6-Fluoro-7-(4-methylaminophenyl)-4-oxo-1-phenyl-4H-[1,3]thiazeto[3,2-a]quinoline-3-carboxylic acid

m.p. ≧ 300 °C

| Elemental analysis ($C_{24}H_{17}FN_2O_3S \cdot 3/4\ H_2O$) | | | |
|---|---|---|---|
| Calcd. (%) | C 64.64 | H 4.18 | N 6.28 |
| Found (%) | C 64.73 | H 3.87 | N 6.32 |

IR $\nu_{max}$ $cm^{-1}$: 3360, 1700, 1600, 1530, 1470

Example 46 6-Fluoro-7-(4-methylaminophenyl)-4-oxo-4H-[1,3]thiazeto[3,2-a]quinoline-3-carboxylic acid

m.p. ≧ 300 °C
Mass spectrum $M^+$ : 356

Example 47 7-(4-Ethylaminophenyl)-6-fluoro-1-methyl-4-oxo-4H-[1,3]thiazeto[3,2-a]quinoline-3-carboxylic acid

m.p. 231 °C (decomp.)

| Elemental analysis ($C_{20}H_{17}FN_2O_3S \cdot 3/4\ H_2O$) | | | |
|---|---|---|---|
| Calcd. (%) | C 60.37 | H 4.69 | N 7.04 |
| Found (%) | C 60.63 | H 4.57 | N 6.90 |

Example 48 Ethyl 7-(4-dimethylaminophenyl)-6-fluoro-8-methoxy-4-oxo-4H-[1,3]thiazeto[3,2-a]quinoline-3-carboxylate

m.p. 216-217 °C (decomp.)

| Elemental analysis ($C_{23}H_{23}FN_2O_4S$) | | | |
|---|---|---|---|
| Calcd. (%) | C 62.43 | H 5.24 | N 6.33 |
| Found (%) | C 62.28 | H 5.16 | N 6.34 |

Example 49 6-Fluoro-8-methoxy-1-methyl-7-(4-methylaminophenyl)-4-oxo-4H-[1,3]thiazeto[3,2-a]quinoline-3-carboxylic acid

m.p. 250-251 °C (decomp.)

| Elemental analysis ($C_{20}H_{17}FN_2O_4S$) | | | |
|---|---|---|---|
| Calcd. (%) | C 59.99 | H 4.28 | N 7.00 |
| Found (%) | C 60.13 | H 4.47 | N 6.93 |

Example 50 6-Fluoro-7-(4-glycylaminomethylphenyl)-8-methoxy-1-methyl-4-oxo-4H-[1,3 thiazeto[3,2-a]-quinoline-3-carboxylic acid hydrochloride

m.p. ≧ 300 °C

| Elemental analysis ($C_{22}H_{20}FN_3O_5S$ • HCl • $H_2O$) | | | |
|---|---|---|---|
| Calcd. (%) | C 49.86 | H 4.75 | N 7.93 |
| Found (%) | C 49.78 | H 4.80 | N 7.54 |

IR $\nu_{max}$ $cm^{-1}$: 2900, 1680, 1590, 1490

Example 51 2-Dimethylamino-2-oxoethyl 7-(4-aminophenyl)-6-fluoro-1-methyl-4-oxo-4H-[1,3]thiazeto[3,2-a]-quinoline-3-carboxylate

m.p. ≧ 300 °C

| Elemental analysis ($C_{22}H_{20}FN_3O_4S$ • 2 $H_2O$) | | | |
|---|---|---|---|
| Calcd. (%) | C 55.33 | H 5.07 | N 8.80 |
| Found (%) | C 55.54 | H 4.85 | N 8.59 |

IR $\nu_{max}$ $cm^{-1}$: 3420, 3330, 1720, 1640, 1600, 1480

Example 52 2-Diethylamino-2-oxoethyl 7-(4-aminophenyl)-6-fluoro-1-methyl-4-oxo-4H-[1,3]thiazeto[3,2-a]-quinoline-3-carboxylate

m.p. ≧ 300 °C
Mass spectrum $M^+$: 470
IR $\nu_{max}$ $cm^{-1}$: 3330, 1725, 1640, 1590, 1530, 1470

Example 53 2-Diisopropylamino-2-oxoethyl 7-(4-aminophenyl)-6-fluoro-1-methyl-4-oxo-4H-[1,3]thiazeto[3,2-a]quinoline-3-carboxylate

m.p. ≧ 300 °C
Mass spectrum $M^+$: 498
IR $\nu_{max}$ $cm^{-1}$: 3440, 3300, 2950, 1720, 1600, 1480

Example 54 Pivaloyloxymethyl 7-(4-aminophenyl)-6-fluoro-1-methyl-4-oxo-4H-[1,3]thiazeto[3,2-a]quinoline-3-carboxylate

m.p. 214-215 °C (decomp.)

| Elemental analysis ($C_{24}H_{23}FN_2O_5S$) | | | |
|---|---|---|---|
| Calcd. (%)<br>Found (%) | C 61.26<br>C 60.84 | H 4.93<br>H 5.13 | N 5.95<br>N 5.75 |

Example 55 7-(4-Aminophenyl)-6-fluoro-1-methyl-4-oxo-4H-[1,3]thiazeto[3,2-a]quinoline-3-carboxylic acid hydrochloride

m.p. 242 ° C (decomp.)

| Elemental analysis ($C_{18}H_{13}FN_2O_3S$ • HCl • $2H_2O$) | | | |
|---|---|---|---|
| Calcd. (%)<br>Found (%) | C 52.63<br>C 52.70 | H 3.90<br>H 3.97 | N 6.82<br>N 6.69 |

Example 56 7-(4-Aminophenyl)-6-fluoro-1-methyl-4-oxo-4H-[1,3]thiazeto[3,2-a]quinoline-3-carboxylic acid 1/2 sulfate

m.p. 238 ° C (decomp.)

| Elemental analysis ($C_{18}H_{13}FN_2O_3S$ • 1/2 $H_2SO_4$ • 3 $H_2O$) | | | |
|---|---|---|---|
| Calcd. (%)<br>Found (%) | C 49.96<br>C 49.69 | H 3.93<br>H 3.80 | N 6.48<br>N 6.48 |

Test Example

The following are the results of pharmacological tests indicting the usefulness of some representative compounds of the invention.

1. Determination of minimal inhibitory concentration of growth (MIC)-1

Method: MIC was determined by the agar plate dilution method in accordance with the standard protocol of Japan Society of Chemotherapy (cf. Chemotherapy 29 (1), 76-79, 1981). Each bacterial culture obtained by 18-hour incubation in a sensitivity assay bouillon medium at 37 ° C was diluted to $10^6$ CFU/ml with the same medium. Using a microplanter, a sensitivity assay agar medium containing the test drug was inoculated with the diluted culture and incubated at 37 ° C for 18 hours. The MIC value was then determined. As reference drugs, 6-fluoro-1-methyl-4-oxo-7-(1-piperazinyl)-4H-[1,3]thiazeto[3,2-a]quinoline-3-carboxylic acid (referred to as control), ciprofloxacin (CPFX) and ofloxacin (OFLX) were used. The results are shown in Table 1.

Table 1 Antibacterial activity against bacteria

| Organism | | MIC ( μ g/ml) | | |
|---|---|---|---|---|
| | | Example 4 | Example 5 | Example 18 |
| Staphylococcus aureus 209-P JC-1 | 1 | 0.012 | 0.025 | 0.012 |
| Staphylococcus aureus Smith | 2 | ≦ 0.006 | ≦ 0.006 | ≦ 0.006 |
| Staphylococcus aureus OWPH 1984 (MRSA) | 3 | ≦ 0.006 | 0.012 | 0.025 |
| Staphylococcus aureus OWPH 2125 (MRSA, PCA$^r$) | 4 | ≦ 0.006 | 0.05 | 0.20 |
| Staphylococcus epider-midis IFO 12993 | 5 | ≦ 0.006 | 0.025 | 0.10 |
| Micrococcus luteus ATCC 9341 | 6 | 0.20 | 0.39 | 6.25 |
| Enterococcus faecalis ATCC 29212 | 7 | 0.05 | 0.10 | 0.39 |
| Bacillus subtilis ATCC 6633 | 8 | ≦ 0.006 | ≦ 0.006 | ≦ 0.006 |
| Escherichia coli KC-14 | 9 | 0.025 | 0.10 | 0.20 |
| Proteus vulgaris HX-19 | 10 | 0.012 | 0.05 | 0.025 |
| Pseudomonas aeruginosa IFO 3445 | 11 | 0.10 | 0.20 | 0.39 |

MRSA: Methicillin-resistant S. aureus

PCA$^r$ : New quinolone-resistant bacteria

Table 1 (continued)

| | MIC (μ g/ml) | | | | |
|---|---|---|---|---|---|
| | Example 19 | Example 21 | Example 24 | Example 25 | Example 26 |
| 1 | 0.012 | 0.05 | 0.012 | ≦ 0.006 | 0.025 |
| 2 | ≦ 0.006 | 0.05 | ≦ 0.006 | ≦ 0.006 | 0.012 |
| 3 | 0.025 | 0.025 | 0.012 | 0.012 | 0.025 |
| 4 | 0.20 | 0.39 | 0.05 | 0.012 | 0.10 |
| 5 | 0.025 | 0.05 | 0.012 | .006 | 0.025 |
| 6 | 6.25 | 3.13 | 0.39 | 0.20 | 0.78 |
| 7 | 0.20 | 0.39 | 0.05 | 0.05 | 0.20 |
| 8 | ≦ 0.006 | 0.025 | ≦ 0.006 | ≦ 0.006 | ≦ 0.006 |
| 9 | 0.10 | 0.78 | 0.20 | 0.05 | 0.78 |
| 10 | 0.012 | 0.39 | 0.10 | 0.025 | 0.39 |
| 11 | 0.78 | 6.25 | 0.39 | 0.20 | 3.13 |

Table 1 (continued)

| | MIC (μ g/ml) | | | | |
|---|---|---|---|---|---|
| | Example 27 | Example 31 | Example 38 | Example 44 | Example 46 |
| 1 | 0.012 | 0.012 | 0.10 | ≦ 0.006 | 0.025 |
| 2 | ≦ 0.006 | ≦ 0.006 | 0.025 | ≦ 0.006 | ≦ 0.006 |
| 3 | ≦ 0.006 | 0.025 | 0.025 | 0.012 | 0.012 |
| 4 | ≦ 0.006 | 0.20 | 0.20 | 0.05 | 0.10 |
| 5 | ≦ 0.006 | ≦ 0.006 | 0.10 | ≦ 0.006 | 0.05 |
| 6 | 0.10 | 1.56 | 0.39 | 0.10 | 0.78 |
| 7 | 0.05 | 0.10 | 0.20 | 0.10 | 0.20 |
| 8 | ≦ 0.006 | ≦ 0.006 | 0.012 | ≦ 0.006 | ≦ 0.006 |
| 9 | 0.10 | 0.025 | 0.39 | 0.10 | 0.10 |
| 10 | 0.012 | ≦ 0.006 | 0.78 | 0.025 | 0.025 |
| 11 | 0.78 | 0.20 | 1.56 | 0.20 | 0.78 |

Table 1 (continued)

| | MIC (μ g/ml) | | | | |
|---|---|---|---|---|---|
| | Example 47 | Example 49 | Example 55 | Example 56 | Control |
| 1 | 0.025 | 0.025 | 0.025 | 0.025 | 0.10 |
| 2 | ≦ 0.006 | ≦ 0.006 | 0.012 | ≦ 0.006 | 0.10 |
| 3 | 0.025 | 0.012 | 0.012 | 0.012 | 0.39 |
| 4 | 0.05 | 0.10 | 0.05 | 0.05 | 12.5 |
| 5 | ≦ 0.006 | 0.012 | 0.012 | 0.012 | 0.20 |
| 6 | 0.39 | 0.20 | 0.39 | 0.20 | 1.56 |
| 7 | 0.10 | 0.025 | 0.10 | 0.05 | 0.39 |
| 8 | ≦ 0.006 | ≦ 0.006 | ≦ 0.006 | ≦ 0.006 | 0.10 |
| 9 | 0.20 | 0.39 | 0.10 | 0.05 | 0.012 |
| 10 | 0.05 | 0.39 | 0.025 | ≦ 0.006 | 0.025 |
| 11 | 1.56 | 3.13 | 0.39 | 0.20 | 0.05 |

Table 1 (continued)

| | MIC (μg/ml) | |
|---|---|---|
| | CPFX | OFLX |
| 1 | 0.20 | 0.39 |
| 2 | 0.20 | 0.20 |
| 3 | 0.39 | 0.39 |
| 4 | 12.5 | 6.25 |
| 5 | 0.39 | 0.39 |
| 6 | 0.39 | 3.13 |
| 7 | 1.56 | 1.56 |
| 8 | 0.05 | 0.10 |
| 9 | 0.025 | 0.05 |
| 10 | 0.025 | 0.05 |
| 11 | 0.20 | 0.78 |

CPFX: Ciprofloxacin
OFLX: Ofloxacin

Against gram-positive bacteria, the compounds of the present invention showed high activities several to scores of times as high as the activity of the reference new quinolone synthetic antimicrobial agents. Furthermore, compared with these reference new quinolone synthetic antimicrobial agents, the compounds of the invention were 10 to scores of times as active against methicillin-resistant Staphylococcus aureus and 30 to hundreds of times as active against new quinolone-resistant and methicillin-resistant Staphylococcus aureus. The compounds further showed sufficient antibacterial activity against gram-negative bacteria.

2. Determination of minimal inhibitory concentration (MIC)-2

The antibacterial activity of the compounds obtained in Example 4, Example 25 and Example 26 of the present invention was assayed by the same procedure as in Test Example 1. As reference drugs, 6-fluoro-1-methyl-4-oxo-7-(1-piperazinyl)-4H-[1,3]-thiazeto[3,2-a]quinoline-3-carboxylic acid (referred to as Control), norfloxacin (NFLX) and tosufloxacin (TFLX) were used. The results are shown in Table 2.

Table 2- Antibacterial activity against new quinolone-resistant and methicillin-resistant Staphylococcus aureus

| Organism | MIC (μg/ml) | | | |
|---|---|---|---|---|
| | Example 4 | Example 25 | Example 26 | Control |
| 1 S. aureus OWPH 1770 | 0.20 | 0.10 | 0.39 | 50 |
| 2 S. aureus OWPH 1773 | 0.78 | 0.20 | 0.39 | 100 |
| 3 S. aureus OWPH 1786 | 1.56 | 0.39 | 0.78 | >100 |
| 4 S. aureus OWPH 1792 | 0.78 | 0.39 | 1.56 | 100 |
| 5 S. aureus OWPH 1793 | 1.56 | 0.39 | 1.56 | >100 |
| 6 S. aureus OWPH 1796 | 0.78 | 0.20 | 0.78 | 25 |
| 7 S. aureus OWPH 1799 | 1.56 | 0.39 | 1.56 | >100 |
| 8 S. aureus OWPH 2124 | 0.78 | 0.39 | 0.39 | 12.5 |
| 9 S. aureus OWPH 2127 | 1.56 | 0.39 | 1.56 | >100 |
| 10 S. aureus OWPH 2128 | 1.56 | 0.39 | 1.56 | >100 |
| 11 S. aureus OWPH 2133 | 0.78 | 0.39 | 1.56 | 25 |
| 12 S. aureus OWPH 2134 | 0.78 | 0.20 | 0.78 | 50 |
| 13 S. aureus Sh 2 | 0.78 | 0.39 | 1.56 | 100 |
| 14 S. aureus Sh 8 | 0.78 | 0.20 | 0.78 | 100 |
| 15 S. aureus Sh 44 | 0.78 | 0.20 | 0.78 | 50 |
| 16 S. aureus Sh 47 | 0.78 | 0.39 | 0.78 | 100 |
| 17 S. aureus Sh 54 | 0.78 | 0.20 | 0.78 | >100 |

Table 2 (Continued)

| | MIC (μ g/ml) | |
|---|---|---|
| | NFLX | TFLX |
| 1 | >100 | >100 |
| 2 | >100 | >100 |
| 3 | >100 | >100 |
| 4 | >100 | >100 |
| 5 | >100 | >100 |
| 6 | >100 | >100 |
| 7 | >100 | >100 |
| 8 | >100 | 12.5 |
| 9 | >100 | >100 |
| 10 | >100 | >100 |
| 11 | >100 | >100 |
| 12 | >100 | >100 |
| 13 | >100 | >100 |
| 14 | >100 | >100 |
| 15 | >100 | >100 |
| 16 | >100 | >100 |
| 17 | >100 | >100 |

NFLX: norfloxacin

TFLX: tosufloxacin

It will be apparent from Table 2 that against new quinolone-resistant and methicillin-resistant S. aureus, the compounds of the present invention are not less than one hundred times as active as the reference new quinolone synthetic antimicrobial drugs.

EFFECTS OF THE INVENTION

It will be seen from the foregoing facts that the compound of the present invention has high antibacterial activity against a variety of bacteria. Particularly against gram-positive bacteria and, inter alia, against methicillin-resistant bacteria and new quinolone-resistant and methicillin-resistant bacteria, the compound of the present invention has by far higher activity than reference drugs and was very effective.

Furthermore, the compound of the invention was very low in toxicity and, therefore, may find broader application than the antibiotic vancomycin. Therefore, the compound of the present invention can be safely used in mammalian animals inclusive of man as a therapeutic drug for systemic infections (respiratory tract infection, urinary tract infection, biliary tract infection, etc.) or local infections.

## Claims

1. A quinolinecarboxylic acid derivative of the following general formula [I] and a physiologically acceptable salt thereof.

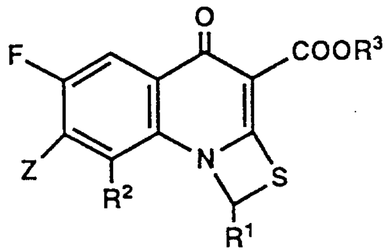

[I]

wherein Z is substituted or unsubstituted phenyl,
$R^1$ is hydrogen, alkyl, or substituted or unsubstituted phenyl,
$R^2$ is hydrogen, alkoxy or halogen, and
$R^3$ is hydrogen, or substituted or unsubstituted alkyl.

2. The quinolinecarboxylic acid derivative and physiologically acceptable salt according to claim 1 wherein Z is phenyl, hydroxyphenyl, alkoxyphenyl, hydroxyalkylphenyl, alkoxyalkylphenyl, nitrophenyl, halophenyl, or phenyl substituted by

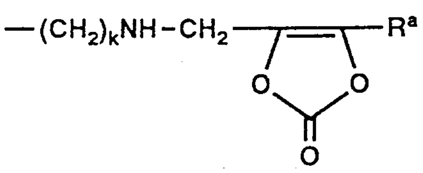

or

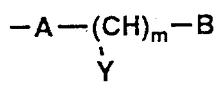

[k is 0 or 1, $R^a$ is alkyl, A is a bond or $-(CH_2)nNHCO-$, n is 0 or 1, Y is hydrogen or hydroxy, m is 0 or 1, -B is $-CH3$ or

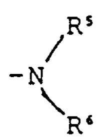

,

wherein $R^5$, $R^6$ may be the same or different and each is hydrogen or alkyl],
$R^1$ is hydrogen, alkyl, phenyl, or halophenyl,
$R^2$ is hydrogen, alkoxy or halogen,
$R^3$ is hydrogen, alkyl, pivaloyloxymethyl, carbamoylalkyl, N-mono- or dialkyl-substituted carbamoylalkyl, or alkyl substituted by

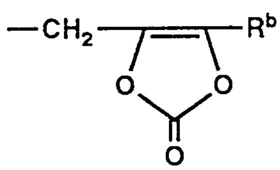

[$R^b$ is alkyl].

3. A compound of the following general formula [II] and a salt thereof.

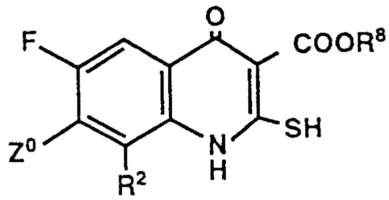

[II]

wherein $Z^0$ is substituted or unsubstituted phenyl,
$R^2$ is hydrogen, alkoxy or halogen,
$R^8$ is alkyl.

# INTERNATIONAL SEARCH REPORT

International Application No PCT/JP91/01327

**I. CLASSIFICATION OF SUBJECT MATTER** (if several classification symbols apply, indicate all) [6]

According to International Patent Classification (IPC) or to both National Classification and IPC

Int. Cl$^5$ C07D513/04, A61K31/47

**II. FIELDS SEARCHED**

Minimum Documentation Searched [7]

| Classification System | Classification Symbols |
|---|---|
| IPC | C07D513/04, A61K31/47 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| A | JP, A, 1-294680 (Nippon Shinyaku Co., Ltd.), November 28, 1989 (28. 11. 89) & EP, A, 315828 | 1-3 |
| A | JP, A, 1-230584 (Nippon Shinyaku Co., Ltd.), September 14, 1989 (14. 09. 89) & EP, A, 315827 | 1-3 |
| A | JP, A, 63-107990 (Nippon Shinyaku Co., Ltd.), May 12, 1988 (12. 05. 88) & EP, A, 249043 & US, A, 4843070 | 1-3 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| December 3, 1991 (03. 12. 91) | December 17, 1991 (17. 12. 91) |
| **International Searching Authority** | **Signature of Authorized Officer** |
| Japanese Patent Office | |